# EUROPEAN PATENT APPLICATION

(11) **EP 1 693 003 A1**
(43) Date of publication of application: **23.08.2006**
(21) Application number: 06250827.0
(22) Date of filing: 16.02.2006
(51) Int. Cl.: A61B 5/103

(54) **Device and method for demonstrating and quantifying skin texture**

(30) Priority: 17.02.2005 US 59797
(71) Applicant: JOHNSON & JOHNSON CONSUMER COMPANIES, INC., Skillman, NJ 08558 (US)
(72) Inventor: Kollias, Nikiforos, Skillman, NJ 08558 (US); Bargo, Paulo Rodriquez, Lawrenceville, NJ 08648 (US)
(74) Representative: Mercer, Christopher Paul

(57) **Abstract**

A device and a method for demonstrating skin texture are disclosed. The device includes a camera having a rotating linear analyzer and a polarized light source. The method includes taking a photograph of an individual using a device comprising a flash lamp having a rotating linear polarizer and a digital camera with a rotating linear analyzer, wherein the camera is positioned at 90 degrees from the flash lamp and the individual is placed such that the illumination angle to its surface normal is from about 45 degrees to about 135 degrees; exporting the photograph to a computer; and making a determination selected from the group consisting of the mean, the median, the intensity distribution and the autocorrelation on a region of interest of the image to demonstrate the texture of the skin.

## Description

### BACKGROUND OF THE INVENTION

### 1. Field of the Invention

The present invention relates to a device and method for demonstrating, quantifying and documenting skin texture. The device is based on polarization imaging using linear polarizers. This device and method are useful for diagnosing where skin needs treatment and to evaluate where the skin has improved as a result of treatment. The method is particularly useful for demonstrating the effectiveness of cosmetic products at changing the skin surface such as increasing skin smoothness and reducing wrinkles and fine lines.

### 2. Description of the Prior Art

Determination of skin surface texture is of extreme importance in the field of dermatology. Such measurements could be used for skin diagnostics and evaluation of therapeutic or cosmetic treatments. Evaluation of skin texture by means of visual and/or touch inspection has been performed for centuries and an experienced professional such as a dermatologist can assess the changes on the skin due to a treatment with great accuracy. With the advances in technology the ability to document skin texture has become a powerful adjunct methodology when evaluating a skin condition. Some technologies can also provide quantitative measurements, which could improve the accuracy of the diagnostic tool or evaluation. Early studies of skin texture were based on profilometry of skin replicas. The major problem with this technology is the artifacts introduced when making the replicas such as bubbles. In general, methods for *in vivo* skin texture measurements have to be non-invasive and non-destructive, hence optical methods are preferred. Recently, three-dimensional (3D) photography (by means of fringe projection or laser scanning) has been established as a very accurate *in vivo* method for texture evaluation and it has been applied in skin evaluation. This method suffers from its complexity, which involves expensive equipment and laborious data collection and analysis.

Polarized light has been utilized in the imaging of various substrates including skin. United States Patent Number 5,742,392 teaches the use of polarized light to visualize subsurface detail of the skin while the skin is viewed through optical elements and filters. The use of polarization imaging with both the source and detector polarizing elements having the same orientation (parallel or perpendicular) has been suggested as a means of enhancing visualization of top layers of the skin. In these applications the surface information due to skin roughness is avoided by spatially filtering the surface reflectance or by implementing an optical window (e.g. adding an optical flat coupled onto the skin with a matching fluid). To our knowledge polarization imaging has not been used to measure skin texture.

### SUMMARY OF THE INVENTION

The present invention provides a device and method for demonstrating, measuring and/or documenting skin texture through the use of a polarization imaging system. The device is composed of a light source with a rotating linear polarizer and an imaging device with a rotating linear analyzer to take high-resolution images of a subject that are stored on a computer. The imaging device is positioned at an angle close to normal to the skin (between ±20 degrees) and the illumination angle is from about 45 degrees to about 85 degrees to a surface normal of the skin to be imaged. Parallel, perpendicular and cross-polarized images are taken and processed to determine the subject's skin texture. Perpendicular images enhance the visualization of surface texture and combination of these images with parallel and/or cross-polarized images can increase the sensitivity of the device. Measurement of skin texture is determined by quantifying the width of the histogram distribution and changes on the distribution shape. Parameters such as the standard deviation and/or the kurtosis can be used to characterize the histogram distribution. The method minimizes the interference of subsurface features and the analysis of the images enable the user to evaluate small changes in skin texture over time or with treatment.

### DETAILED DESCRIPTION OF PREFERRED EMBODIMENTS

In one embodiment, the present invention provides a device including an imaging device having a rotating linear analyzer oriented perpendicular to a plane of incidence and a light source having a rotating linear polarizer oriented perpendicular to a plane of incidence. Any imaging device having a rotating linear analyzer is suitable for use in the present invention. Suitable imaging devices include, but are not limited to, a camera, a digital camera, a scanner, and a charge coupled device detector. A digital camera having a rotating linear analyzer is preferred. One example of a suitable digital camera is a Nikon brand D1X digital camera. The light source utilized in the present invention includes a light source having a rotating linear polarizer. Any light source such as CW lamps, flash lamps, LEDs or lasers with a polarizer may be utilized. One example of a suitable flash lamp is a Broncolor brand Picolite flash lamp. Broadband (e.g. white light) or narrow band light sources can be used. White light sources are preferred for qualitative visualization of skin texture whereas the preferred wavelengths for measurement of skin texture are the shorter wavelengths (e.g. UV and blue) since they provide better structural resolution and shallower penetration into the skin. The wavelength selection can be achieved by either using a narrow band light source or by filtering the light collected by the camera.

The imaging device is positioned at an angle between about 45 degrees and 135 degrees to the polarized light source. Preferably the imaging device is positioned at 90 degrees from the light source. A surface to be measured is positioned such that the illumination angle to the surface normal may range from about 45 degrees to about 85 degrees and that the imaging device views the surface with an angle from about -20 degrees to about 20 degrees to the surface normal. Most preferably, the illumination and the detection angle to the surface normal are 70 degrees and 0 degrees, respectively. Images taken with this device will demonstrate the texture of the skin.

In a second embodiment of the present invention, the device is attached to a computer. In this embodiment, the texture of the skin may be quantified. The method for quantifying skin texture includes taking an image of an object utilizing the device described above, exporting the image from the imaging device to a computer, and quantitatively analyzing the texture using the blue wavelength of the images.

In this method of the present invention, the imaging device is preferably positioned at 90 degrees from the flash lamp and a subject is placed such that the illumination angle to its surface normal is from about 45 degrees to about 85 degrees. Preferably, the illumination angle to its surface normal is 70 degrees.

Parallel, perpendicular and cross-polarized images are taken and exported into a computer. Parallel images are taken with both the polarizer and the analyzer oriented with the electric field parallel to the plane of incidence. The plane of incidence is defined between the source, detector and the subject and for the present invention is horizontal. Perpendicular images are taken with both the polarizer and the analyzer oriented with the electric field perpendicular to the plane of incidence. Cross-polarized images are taken with the polarizer and the analyzer having opposite orientation (either parallel / perpendicular or perpendicular / parallel). At the illumination and collection angles used in the present invention the perpendicular image enhances the surface texture and can visually demonstrate the quality and changes in texture of the skin of an individual. Furthermore, a computer program is used to determine the histogram of a region of interest ("ROI") and make a determination selected from the group consisting of its mean, median, standard deviation, intensity distribution, skewness, autocorrelation function, kurtosis and combinations thereof. The shape of the histogram may be used to quantify the skin texture. Narrower histogram distribution indicates a smoother surface within the ROI. The standard deviation and/or the kurtosis can be used as parameters to characterize the histogram distribution. Higher values of standard deviation or kurtosis (hence larger histogram distributions) indicate greater roughness. This analysis can be performed on either the perpendicular image alone or in combined images (e.g. perpendicular minus cross-polarized or perpendicular minus parallel). Other methods of analysis can be performed by calculating the autocorrelation function of the ROI or by implementing roughness analysis models such as BRDF models.

The method is useful for evaluating the skin condition and for demonstrating the effectiveness of cosmetic products. For example, images may be taken prior to and after application of an anti-wrinkle cream or a film-forming product. These products increase the smoothness of the skin texture, which can be visualized with the perpendicular polarized images and then quantified. The same is true for dermabrasion products.

### Example 1

A device was assembled with a Broncolor Picolite flash lamp as the light source positioned at zero degrees to illuminate the front of the subject's face. The lamp was driven by a Broncolor Nano A4 power supply and it was coupled to a Colin S.A. diffuser to produce a uniform beam. An Edmund Optics linear sheet polarizer was attached to the flash lamp through a Cokin S.A. P series holder. The polarizer was attached such that it could be easily removed to produce unpolarized light or rotated to produce perpendicular or parallel-polarized light.

The subject was positioned with his head on a custom made jig composed of a chin rest and a head restrainer such that repositioning of the head could be obtained.

A digital high resolution SLR color camera (D1X, Nikon, Japan) with a macro zoom lens was positioned at 90 degrees from the flash, to record images from the side of the subject's face. A linear polarizer was attached to the camera's lens through a holder. Like the source polarizer, the camera polarizer (or analyzer) could also be removed or rotated.

A laptop computer with custom made software based on IDL controlled the camera and stored the digital images through an IEEE 1394 (firewire) communication port. Distances between the source and the subject and between the subject and the camera were equal to 75 cm. The source power supply was set to 6.6 V. The camera parameters were set as follows: shutter time = 1/125, sensitivity = 320, aperture = 16.0 and white balance = sunny. The lens was set to 50 mm focal point when full face images were taken and up to 105 mm for detailed images.

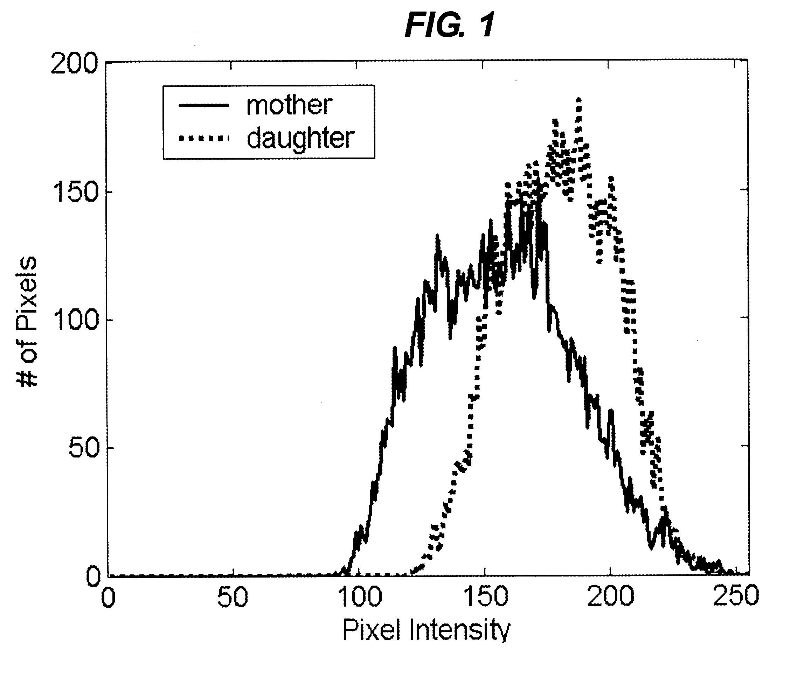

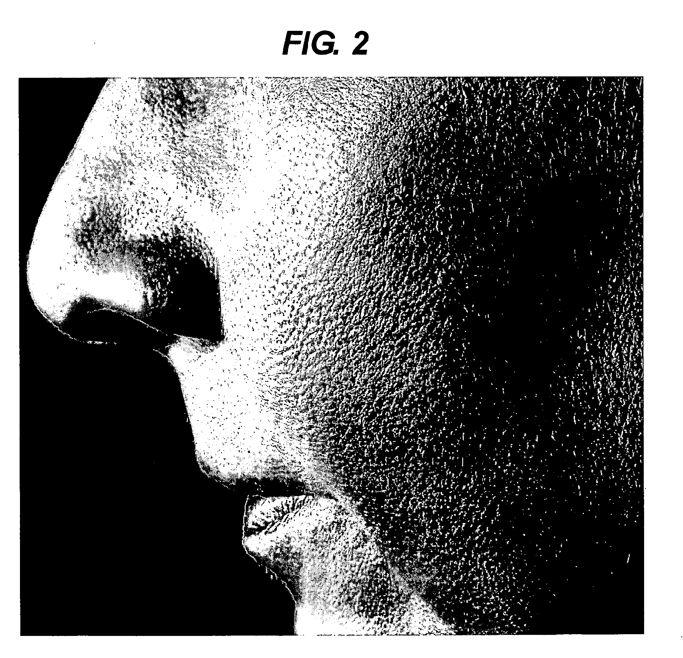

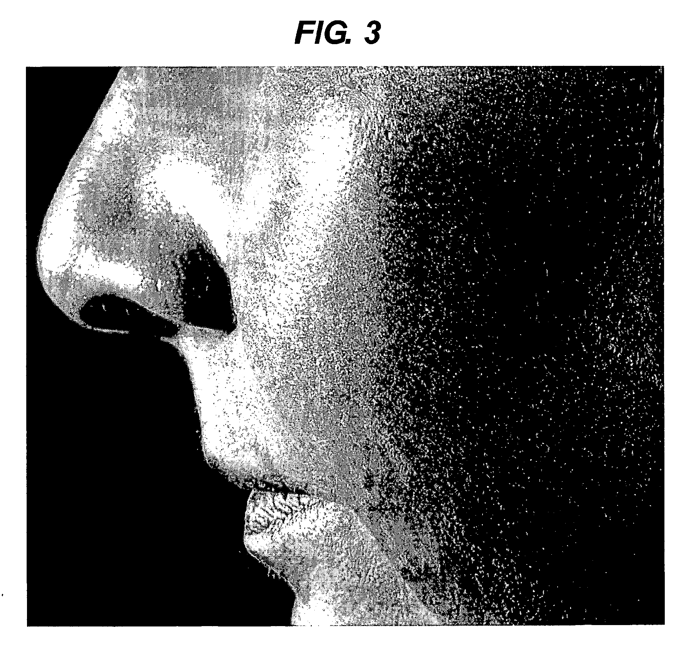

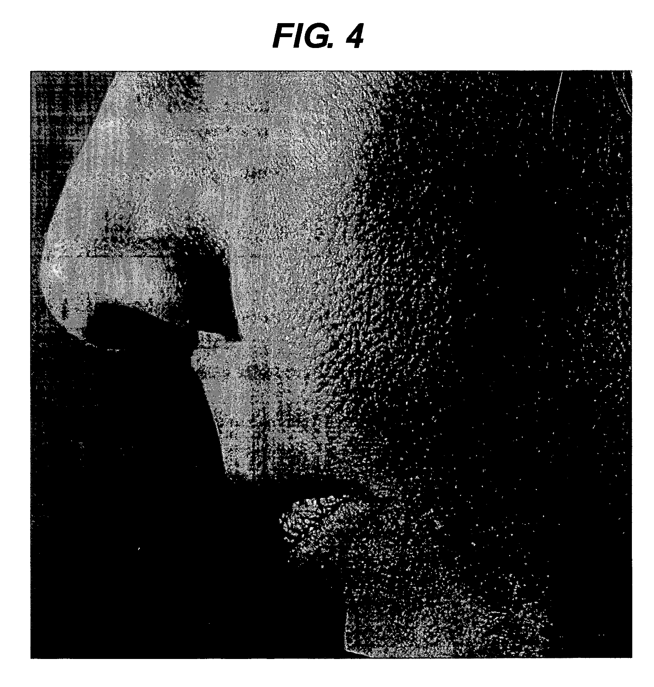

Experiments were performed to demonstrate that the method of this invention distinguishes significant texture variations between old and young skin. Figure 1 shows the perpendicular images histogram of a mother in her late forties and her teenage daughter. The histogram of the marked ROI of the perpendicular and the cross-polarized images for both subjects are shown. The ratio of width of the histogram in the perpendicular image to the width of the histogram of the cross-polarized image for the mother is larger than the same ratio for the daughter, which represents the difference between the surface roughness. Skin smoothing products such as bovine serum albumine were applied to the face of a subject. Before (Figure 2) and after (Figure 3) images were taken and compared. The effect of the bovine serum albumin on smoothing the skin texture was visible in the generated images. Figure 4 is a comparative photo taken under conventional conditions.

A microdermabrasion cream was applied to the facial skin of a subject utilizing a microdermabrasion device. Before and after images were compared. The after image clearly appeared to have a more smooth skin texture.

## Claims

1. A device comprising:
an imaging device having a rotating linear polarizer oriented perpendicular to a plane of incidence; and
a light source having a rotating linear polarizer oriented perpendicular to a plane of incidence,
wherein the imaging device is positioned from about 45 degrees to about 135 degrees to the light source and an illumination angle ranges from about 45 degrees to about 85 degrees to a surface normal of an object to be imaged.

2. The device according to Claim 1 wherein the imaging device is a digital camera and the angle between the camera and the light source is 90 degrees.

3. The device according to Claim 2 wherein the illumination angle to its surface normal is 70 degrees.

4. The device according to Claim 1 further comprising a computer, wherein the imaging device is connected to the computer.

5. A method for demonstrating skin texture comprising:
taking an image of an individual using a device comprising a flash lamp having a rotating linear polarizer oriented perpendicular to a plane of incidence and a digital camera with a rotating linear analyzer oriented perpendicular to a plane of incidence, wherein the camera is positioned from about 45 degrees to about 135 degrees from the flash lamp and the individual is placed such that the illumination angle to its surface normal is from about 45 degrees to about 85 degrees;
exporting the image to a computer; and
making a determination selected from the group consisting of the mean, the median, the intensity distribution, the standard deviation, the kurtosis, the autocorrelation function and combinations thereof in the blue wavelengths on a region of interest of the image to document and quantify the texture of the skin.

6. The method according to Claim 5 wherein the angle between the camera and the light source is 90 degrees.

7. The method according to Claim 5 wherein the illumination angle to its surface normal is 70 degrees.

8. The method according to Claim 5 wherein the calculations are based on a computer generated histogram.
